(19) **Europäisches Patentamt / European Patent Office / Office européen des brevets**

(11) **EP 1 505 393 B1**

(12) **EUROPEAN PATENT SPECIFICATION**

(45) Date of publication and mention
of the grant of the patent:
**31.12.2014 Bulletin 2015/01**

(51) Int Cl.:
*G01N 33/543* (2006.01)     *G01N 33/92* (2006.01)
*B01D 39/00* (2006.01)

(21) Application number: **04254302.5**

(22) Date of filing: **16.07.2004**

(54) **Dry analytical element for high-density lipoprotein cholesterol quantification**

Vorrichtung zur Quantifizierung von HDL-Cholesterin

Dispositif de test permettant d'essayer le cholesterol HDL

(84) Designated Contracting States:
**AT BE BG CH CY CZ DE DK EE ES FI FR GB GR HU IE IT LI LU MC NL PL PT RO SE SI SK TR**

(30) Priority: **17.07.2003 US 488101 P**

(43) Date of publication of application:
**09.02.2005 Bulletin 2005/06**

(73) Proprietor: **ORTHO-CLINICAL DIAGNOSTICS, INC.**
**Rochester, NY 14626-5101 (US)**

(72) Inventors:
• **DiMagno, Theodore John**
  **Penfield, NY 14526 (US)**
• **Arter, Thomas Charles**
  **Rochester, NY 14612 (US)**
• **Chambers, Deborah Lynn**
  **Hilton, NY 14468 (US)**
• **Silva, David Paul, Jr.**
  **Rochester, NY 14617 (US)**
• **Vavra, Karen J.**
  **Rochester, NY 14617 (US)**

(74) Representative: **Bullett, Rachel Margaret et al**
  **Carpmaels & Ransford LLP**
  **One Southampton Row**
  **London WC1B 5HA (GB)**

(56) References cited:
EP-A- 0 699 767     EP-A- 0 877 251
EP-A- 0 952 451     EP-A- 1 342 792
EP-A- 1 357 383     WO-A- 02/38800
US-A- 5 135 716     US-A- 5 215 886

• PATENT ABSTRACTS OF JAPAN vol. 017, no. 412 (P-1583), 30 July 1993 (1993-07-30) & JP 05 080056 A (KONICA CORP), 30 March 1993 (1993-03-30)

**Description**

Background

The invention relates generally to a dry analytical element used to quantify high-density lipoprotein cholesterol (HDLC) in fluid samples. More specifically, the invention relates to a support containing specific reagent layers, which improve HDLC selectivity to create a single-step, dry slide assay to quantify HDLC in the presence of other lipoproteins.

Lipoproteins can be categorized as high-density lipoprotein (HDL), low-density lipoprotein (LDL), very low-density lipoprotein (VLDL) or chylomicron (CM). It is known that HDLC is related to the removal of cholesterol accumulation from tissues including arterial walls. Therefore, HDLC is measured as a negative risk factor for various types of arteriosclerosis such as coronary arteriosclerosis. The HDLC level in blood is a useful index for the precognition of arteriosclerosis.

There is continuing need in medical practice and research, and in analytical and diagnostic procedures, for rapid and accurate determinations of chemical and biological substances that are present in biological fluids. Specifically, the quantity of HDLC in serum or plasma must be determined rapidly and accurately for diagnosis, monitoring, and treatment of cardiovascular disease or disease risk.

Some conventional methods for quantifying HDLC rely on various techniques for separating HDL from non-high density lipoproteins (non-HDLs), including LDL, VLDL, and CM, followed by a quantification of cholesterol contained in the HDL fraction. One such example is the VITROS HDLC assay, in which non-HDL is precipitated with dextran sulfate and removed by centrifugation or magnetic separation. The HDLC in the supernatant is then quantified using a cholesterol dry analytical element.

More recently, methods for direct HDLC solution assays have been developed. A direct high-density lipoprotein cholesterol assay utilizes preferential selectivity for HDL over non-high density lipoproteins. Many well-known solution methods have been shown to improve assay specificity for HDLC including non-HDL precipitation methods (1,2,3,4,5), immuno-inhibition (6,7), selective surfactants (8,9,10,11), catalase elimination (12), and polyethylene glycol (PEG) cross-linked cholesterol esterase (CEH) (13).

Unfortunately, the solution methods typically use multiple reagent additions or separation steps that do not adapt well to an all-inclusive, single-step, dry slide assay. Moreover, precipitation methods and selective surfactant methods alone do not yield sufficient HDLC selectivity in the dry, multilayered slide format. None of the well-known HDLC methods are amenable to or yield sufficient selectivity in this format, which would be desirable for rapid and accurate diagnosis of HDLC quantity. In view of the need to create a single-step, dry slide assay, a novel method is needed to improve HDLC selectivity for the assay.

Summary of the Invention

The present invention provides a format for a dry analytical element designed for the rapid and accurate quantification of high-density lipoprotein cholesterol. The assay does not require sample pretreatment to remove non-HDL lipoproteins prior to sample application. The element comprises a support containing a first enzyme, a cholesterol ester hydrolase, to hydrolyze cholesterol esters, a second enzyme, cholesterol oxidase, to release hydrogen peroxide from cholesterol, and a third enzyme, horseradish peroxidase, to oxidize a leuco dye that is read at 670 nm. The HDLC selectivity arises from 1) precipitation and trapping of non-HDL lipoproteins in the spread layer, 2) selective solubilization of HDL while maintaining non-HDL-polyanion complexes intact with a unique surfactant in the spread layer, and 3) selective hydrolysis of the ester bond by a unique selective cholesterol ester hydrolase (CEH) in the polymer receptor layer. Improved precision can be obtained through the use of a polyethylene glycol as a matrix for the CEH-containing layer. Significant interference from hemolyzed patient samples has been eliminated through the selection of a polymeric matrix for the dye-containing layer.

The dry analytical element comprises one or more layers coated on a support. The one or more layers comprise a non-high density lipoprotein precipitant-phosphotungstic acid, a high-density lipoprotein selective surfactant, and a high-density lipoprotein selective cholesterol ester hydrolase enzyme. The high-density lipoprotein selective surfactant is preferably EMULGEN B-66 or EMULGEN A-90 (polyalkylene derivatives). The high-density lipoprotein selective cholesterol ester hydrolase enzyme is preferably *Candida rugosa* lipase. The one or more additional layers further include polyethylene glycol and dimedone (5,5-Dimethyl-1,3-Cyclohexanedione) to improve precision. The support comprises transparent material from a group consisting of polystyrene, polyesters, polycarbonates, and cellulose esters.

In one embodiment, the dry analytical element above comprises a support with multiple layers coated in the following order, top down to the support:

(i) a washcoat layer comprising phosphotungstic acid, a non-high density lipoprotein precipitant,
(ii) a porous spread layer comprising a high-density lipoprotein selective surfactant,

(iii) an adhesion layer,
(iv) a polymer receptor layer comprising a high-density lipoprotein selective cholesterol ester hydrolase enzyme,
(v) a dye sublayer comprising a polymer, and
(vi) a gel layer comprising binder materials.

The high-density lipoprotein selective surfactant is preferably EMULGEN B-66 or EMULGEN A-90 (polyalkylene derivatives). The high-density lipoprotein selective cholesterol ester hydrolase enzyme is preferably *Candida rugosa* lipase. The polymer receptor area includes polyethylene glycol. The dye-sublayer includes polyethylene glycol or polyacrylamide co-polymer. The receptor layer comprises dimedone (5,5-dimethyl-1,3-cyclohexanedione). The support comprises transparent material from a group consisting of polystyrene, polyesters, polycarbonates, and cellulose esters. The dye layer or the gel layer includes a leuco dye (2-(3,5-dimethoxy-4-hydroxyphenyl)-4,5-bis(4-dimethylaminophenyl)Imidazole. The gel layer contains horseradish peroxidase. The polymer receptor layer contains cholesterol oxidase from Cellulomonas.

Detailed Description of the Drawings

Figure 1 is a graph of HDLC and LDLC kinetics with a beef pancreas cholesterol esterase (CEH) enzyme source. Human HDL test fluids are shown in solid lines. Human LDL test fluids are shown in dashed lines.

Figure 2 is a graph of HDLC and LDLC kinetics with a porcine pancreas CEH enzyme source. Human HDL test fluids are shown in solid lines. Human LDL test fluids are shown in dashed lines.

Figure 3 is a graph of HDLC and LDLC kinetics with a *Candida rugosa* lipase enzyme source. Human HDL test fluids are shown in solid lines. Human LDL test fluids are shown in dashed lines.

Figure 4 is a graph of HDLC and LDLC kinetics with a Denka CEH enzyme source. Human HDL test fluids are shown in solid lines. Human LDL test fluids are shown in dashed lines.

Figure 5 is a plot of the main effects on LDL cross-reactivity for the polyethylene glycol and dimedone (5,5-dimethyl-1,3-cyclohexanedione).

Figure 6 is a graph of Human HDL test fluid kinetics with a polyacrylamide co-polymer (IMnAg) in the polymer receptor layer. Human HDL test fluids from 0 - 75 mg/dL are shown in solid lines. Human HDL test fluids from 105 -150 mg/dL are shown in dashed lines.

Figure 7 is a graph of Human HDL test fluid kinetics with polyethylene glycol in the polymer receptor layer. Human HDL test fluids from 0 - 75 mg/dL are shown in solid lines. Human HDL test fluids from 105 - 150 mg/dL are shown in dashed lines.

Figure 8 is a main effects plot for linearity with the IMnAg vs. PEG in the polymer receptor layer.

Figure 9 is a main effects plot for pooled standard deviation precision with human HDL test fluids.

Detailed description of the invention

The present invention discloses an assay for the determination of HDLC using a dry analytical element. Dry analytical elements useful for the assay of liquids can be prepared according to the teachings of U.S. Patent No. 3,992,158 (14) and U.S. Patent No. 4,357,363 (15), the contents of which are incorporated herein.
Briefly described, the analytical element of this invention comprises one or more layers coated on a suitable support (Tables 1, 2, and 3). Preferably, the reagents are coated in distinct reagent layers as shown in Table 13.
The support can be any suitable dimensionally stable, and preferably non-porous and transparent material which transmits light of a wavelength between about 200 nm and about 900 nm. Useful support materials include polystyrene, polyesters, polycarbonates, and cellulose esters (e.g. cellulose acetate), but this is not an inclusive list and other suitable materials may be used.
The reagent layers coated on the support contain the elements of the detection and quantification system. These elements are dispersed in one or more synthetic or natural binder materials, such as gelatin or other naturally-occurring colloids, as well as different synthetic hydrophilic polymers such as poly(acrylamide), poly(vinylpyrrolidone), poly(ethylene glycol), copolymers of the above, and polymers or co-polymers to which cross-linkable monomers have been added.

The reagent layers may contain a buffer. The buffer may be in any or all layers of the element, or it may be in a separate layer devoid of other reagents. Different buffers may be used in separate layers of the element.

Several surfactants such as OLIN-10G and ZONYL FSN may be optionally included in one or more reagent layers. Several different cross-linking agents are also optional, such as bisvinylsulfanylmethane, glutaraldehyde, etc.

The element may be provided with a porous, reflective spreading layer to uniformly distribute the liquid test sample over the element. The spreading layer may contain reagents, buffers, surfactants, and other components. Materials for use in spreading layers are well known in the art of making dry analytical elements as disclosed, for example, in U.S. Patent No. 4,258,001 (16) and the above cited patents. Useful spreading layers can be prepared from fibrous materials, polymeric compounds, and beads. Particularly useful spreading layers comprise barium sulfate or titanium dioxide.

Other optional layers, e.g. subbing layers, can be included if desired. The layers of the element can contain a variety of other desirable but optional components, including surfactants, thickeners, buffers, hardeners, antioxidants, bacteriostats, coupler solvents, and other materials known in the art.

Changes in the element can be detected with a suitable spectrophotometric apparatus using generally known procedures disclosed, for example, in U.S. Patent No. 3,992,158 (14) and U.S. Patent No. 4,357,363 (15).

Materials and Conditions

As used herein, the terms described below apply to the following examples. The following examples are meant to illustrate, not limit the present invention.

Buffers were used in concentrations ranging from 2 - 16 $g/m^2$ for a preferred pH range of 5.5 - 7.5. Buffer examples include BES (N,N-bis[2-Hydroxyethyl]-2-aminoehatnesulfonic acid), TES (N-tris[Hydroxymethyl]mehtyl-2-aminoethanesulfonic acid), MES (2-[N-Morpholino]ethanesulfonic acid), and Phosphate.

Dyes were used in concentrations of 0.1 - 1.0 $g/m^2$. A dye example is leuco dye (2-(3,5-dimethoxy-4-hydroxyphenyl)-4,5-bis(4-dimethylaminophenyl)Imidazole. Stabilizer was used in concentrations of 0.05 - 5.5 $g/m^2$. A stabilizer example is dimedone (5,5-Dimethyl-1,3-Cyclohexanedione).

The concentrations of active surfactants were within the 0.5 - 8.0 $g/m^2$ range. Surfactant examples include EMULGEN B-66 (polyoxyalkylene derivative, commercially available from KAO Corporation) and EMULGEN A-90 (polyoxyalkylene derivative, commercially available from KAO Corporation).

Phosphotungstic acid (PTA) concentrations were within 1 - 5 $g/m^2$. An example is phosphotungstic acid sodium salt (P6395 from Sigma). Divalent metal concentrations ranged within 0 - 2.3 $g/m^2$. A divalent metal example includes $MgCl_2$.

Polyethylene glycol was used in concentrations of 1 - 10 $g/m^2$. Polyethylene glycol examples include MW 3,400 (202444 from Aldrich Chemical), MW 8,000 (P4463 or P2139 from Sigma Chemical) and MW 20,000 (813003 from Aldrich Chemical). Poly(acrylamide) co-polymer (IMnAg) was used in concentrations between 0.2 - 0.8 $g/m^2$.

COD - Cholesterol oxidase was used in concentrations of 4 - 16 $kU/m^2$. An example is a microorganism from Cellulomonas (CON-II from Asahi Chemical Industry).

CEH - Cholesterol esterase was used in a concentration range of 2 - 28 $kU/m^2$. Examples include lipase from *Candida rugosa* (Lipase II from Genzyme) and Cholesterol ester hydrolase (Denka Corporation).

AAO - Ascorbic acid oxidase was used in concentrations of 2 - 8 $kU/m^2$. An example is zucchini squash ascorbic acid oxidase (Calzyme).

POD - Peroxidase was used in concentrations a range of 5 - 55 $kU/m^2$. This enzyme comes from horseradish.

3-Amino-1,2,4-triazole was used in a concentration range of 0.1 - 10 $g/m^2$. Examples include HCl salt (A8056 from Sigma Chemical) and HCl salt (A8,160-9 from Aldrich Chemical).

EXAMPLE 1

Example Formats of the Thin Film Element

Three example formats of the thin film element used in this evaluation are shown in Tables 1, 2 and 3. The locations of the enzymes and other reactive ingredients may be placed in a variety of other positions within the layers of the thin film element. However, a preferred embodiment of the present invention includes a non-HDL precipitant (PTA), an HDL selective surfactant, and an HDL selective CEH . Each format of the thin film element contains individual layers, which are represented by the various schematic arrangements. In referring to the arrangements, each layer is numerically designated with '-01' representing the bottom gel layer. Successive numbers represent each additional layer with the washcoat (top layer) designated as '-06' (in a six layer arrangement).

TABLE 1 - Example (1) of a Multilayer Thin Film Element for Quantification of HDLC.

| PTA/MgCl$_2$/Surfactant |
|---|
| BaSO$_4$ Spreadlayer/Surfactant |
| I-100 Adhesion |
| Gel/COD/CEH |
| Gel/Dye/POD |

TABLE 2 - Example (2) of a Multilayer Thin Film Element for Quantification of HDLC

| PTA/MgCl$_2$ |
|---|
| BaSO$_4$ Spreadlayer/Surfactant |
| I-100 Adhesion |
| PEG/COD/CEH |
| Gel/Dye |
| Gel/Dye/POD |

TABLE 3 - Example (3) of a Multilayer Thin Film Element for Quantification of HDLC

| PTA/MgCl$_2$ |
|---|
| BaSO$_4$ Spreadlayer/Surfactant |
| I-100 Adhesion |
| PEG/COD/CEH |
| IMnAg or PEG/Dye |
| Gel/POD |

EXAMPLE 2

Phosphotungstic Acid

It has been known for many decades (17,18,19) that precipitating agents can be used to selectively eliminate non-HDL to enable the development of an HDLC assay that does not require ultra-centrifugation. These precipitating reagents were developed for liquid assays. Their use in dry slide elements was also investigated. The three most common non-HDL precipitating reagents, dextran sulfate (DS), phosphotungstic acid (PTA), and polyethylene glycol (PEG) were evaluated in the thin-film slide. The precipitating reagents were added in the final pass washcoat ('-05' or '-06' depending on format) layer so that they would be on the surface of the slide and interact with the non-HDL lipoproteins spotted on the slide in the initial time frame to enable the largest possible interaction time for precipitation in the thin-film element. Early attempts with DS up to concentrations of 4 g/m$^2$ and with PEG up to 10 g/m$^2$ proved unsuccessful in the thin-film element. PTA, however, showed some specificity enhancement at washcoat concentrations of 2 g/m$^2$.

## EQUATION 1 – Calculation of LDL Cross-reactivity

$$\frac{[\mathrm{Pr}\,ed\_HDLC]}{[LDLC\_Conc]} \times 100 = \%Cross-reactivity$$

The contribution to HDL selectivity by the PTA/MgCl$_2$ can be evaluated in optimized coatings with and without the PTA/MgCl$_2$ washcoat. HDL selectivity can be measured in relation to the "%LDL cross-reactivity" defined in Equation 1, where [Pred_ HDLC] is the sample concentration of LDL analyte that the HDLC thin-film assay incorrectly predicts as HDLC and [LDLC_Conc] is the true human LDL cholesterol analyte concentration contained in the sample. The illustrated format consisting of a '-01' dye and '-03' PEG Polymer (Table 4) was tested with one sample containing PTA/MgCl$_2$ (PTA) in the washcoat and a second sample without PTA/MgCl$_2$ (No PTA) in the washcoat. The overall reactivity of both the human HDL and LDL test fluids are higher in the No PTA washcoat formula. The relative HDLC selectivity is increased in the PTA washcoat formula. The data in Table 4 shows that the contribution of the PTA to the HDLC selectivity in this format is approximately 22% (32.3% - 10.4%).

TABLE 4 - Effect of PTA/MgCl$_2$ on the LDL Cross-Reactivity

| Washcoat | LDL Conc. (mg/dL) | Pred HDL (mg/dL) | % Cross -Reactivity |
|---|---|---|---|
| PTA | 75 | 7.79 | 10.4% |
| No PTA | 75 | 24.23 | 32.3% |

Similar results were obtained in the format with PEG in the '-02' and '-03' layers (Table 3). The PTA contribution to HDLC selectivity was found to be 24.5% (Table 5 (26.3% - 1.8%)). Both of these tests used slightly different formats but consistently showed that PTA contributes an approximate 23% increase in HDLC selectivity compared to the selectivity provided from any other known source.

TABLE 5 - Effect of PTA/MgCl$_2$ on the LDL Cross-Reactivity with the milled dye

| Washcoat | LDL Conc. (mg/dL) | Pred HDL (mg/dL) | % Cross-Reactivity |
|---|---|---|---|
| No Washcoat | 75 | 19.70 | 26.3% |
| PTA/MgCl$_2$ Washcoat | 75 | 1.36 | 1.8% |

EXAMPLE 3

Effect of a Selective CEH on HDLC Specificity

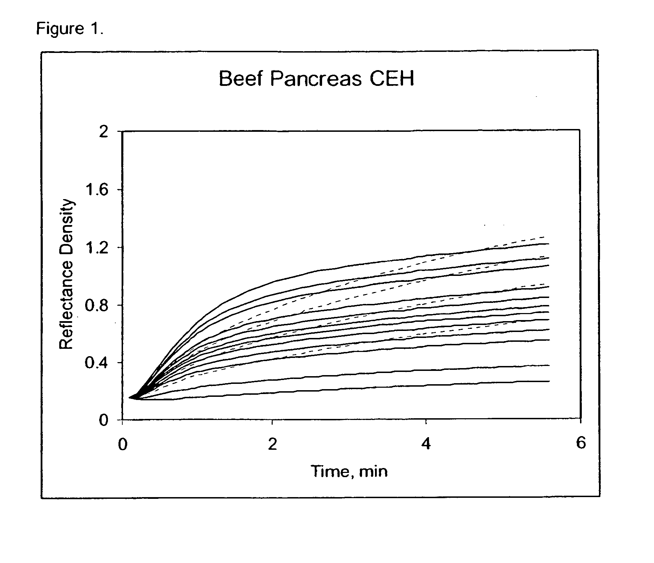

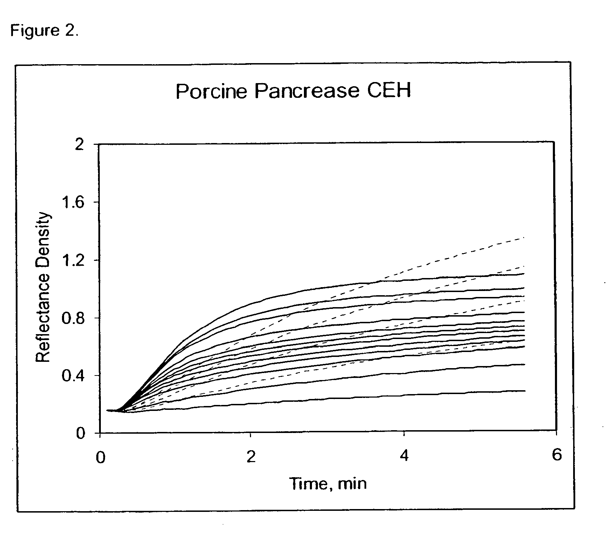

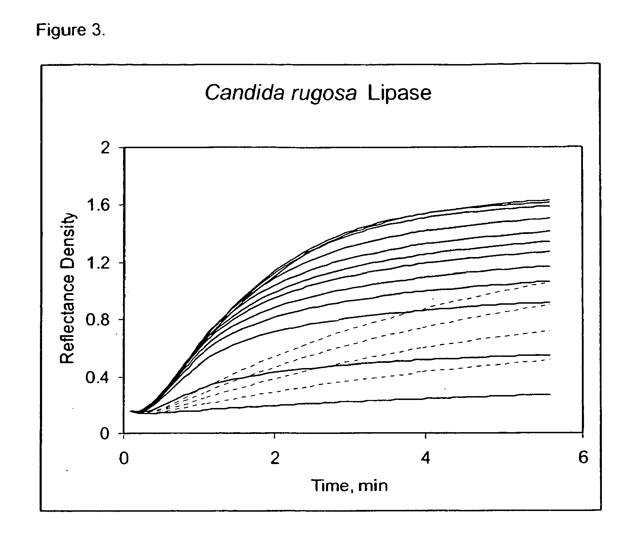

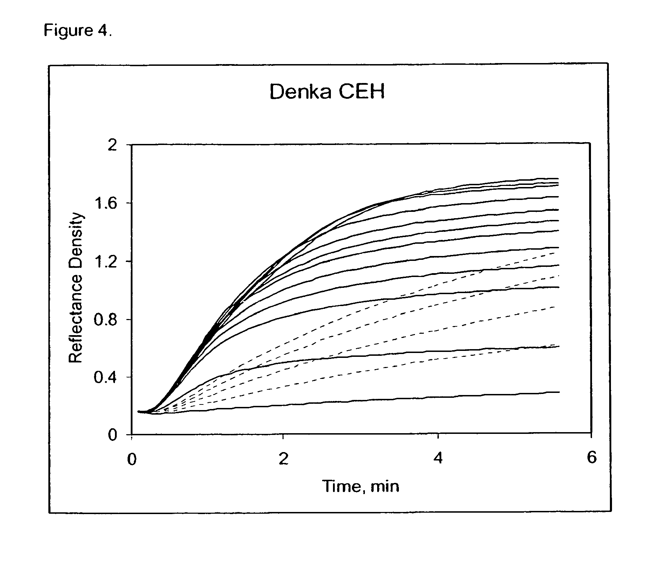

Early development testing showed that various cholesterol esterase (CEH) enzyme sources impacted the overall HDLC selectivity of the assay. Available unique CEH and lipase enzyme sources from current enzyme vendors were acquired for screening purposes and coated in the direct HDL thin-film element. LDL Cross-Reactivity for the best and worst CEH sources shows a substantial HDLC selectivity difference in both the raw kinetics (see Figures 1,2,3,4) and the calculated cross-reactivity (Table 6). From this data, the overall contribution to the HDLC selectivity from the selective CEH alone is approximately 35% (50.7% (Bovine Pancreas) -15.7% (*Candida rugosa* Lipase)), representing a substantial contribution to HDLC selectivity. Furthermore, this data also shows that the *Candida rugosa* lipase enzyme is more selective than the Denka CEH, and considerably better than either pancreas CEH sources. Other screened microorganism CEH sources showed HDLC selectivity within these two extremes.

TABLE 6 - Effect of CEH Enzyme Source on the LDL Cross-Reactivity

| CEH Source | LDL Conc. (mg/dL) | Pred on HDL Web (mg/dL) | %Cross-Reactivity |
|---|---|---|---|
| Denka CEH | 75 | 14.41 | 19.2% |
| Porcine Pancreas CEH | 75 | 29.71 | 39.6% |
| Bovine Pancreas CEH | 75 | 38.01 | 50.7% |
| *Candida rugosa* Lipase | 75 | 11.76 | 15.7% |

EXAMPLE 4

Effect of a Selective Surfactant on HDLC Specificity

Two different identified surfactants, EMULGEN B-66 surfactant, and EMULGEN A-90 surfactant (both are polyoxyalkylene derivatives produced by KAO corporation), show high selectivity for HDL over non-HDL. Other surfactants from KAO Corporation have also shown some intermediate selectivity, but at a lower level than the preferred HDL selective surfactants. Other surfactants, such as TRITON X-100 (TX-100), have lacked any specificity for HDL. The substitution of TX-100 surfactant in the assay caused a complete loss in HDLC specificity (Table 7). The change to a non-selective surfactant indicates a 68% increase in LDL cross-reactivity.

TABLE 7 - Effect of Surfactant on the LDL Cross-Reactivity

| Surfactant | Normalized HDLC Selectivity | Prediction on coating of 75 mg/dL LDL | % Cross-reactivity |
|---|---|---|---|
| EMULGEN A-60 | 0.36 | 66.1 | 88.1% |
| EMULGEN B-66 | 1.00 | 24.1 | 32.1% |
| EMULGEN A-90 | 0.88 | 27.4 | 36.5% |
| EMULGEN 109P | 0.33 | 73.2 | 97.6% |
| EMULGEN 220 | 0:32 | 74.3 | 99.1% |
| TRITON X-100 | 0.30 | 74.6 | 99.4% |

This distinction partially derives from TX-100's ability to completely solubilize the precipitated non-HDL complexes. An additional unique feature of EMULGEN B-66 and EMULGEN A-90 is their compatibility with PTA precipitated non-HDL. Both surfactants do not resolubilize the PTA-MgCl$_2$-non-HDL complexes. Both the inherent HDL selectivity and the lack of solubilization of PTA precipitated non-HDL complexes are essential properties of EMULGEN B-66 and EMULGEN A-90 required in the single step direct HDLC dry slide assay. Whereas, the TX-100 surfactant offsets any specificity gained from PTA precipitation in the direct HDLC dry slide assay.

Therefore, the 68% increase in selectivity can be split between the effect from the selective surfactant, the effect from the selective CEH, and the effect of the PTA. As shown above, the CEH contribution to selectivity is -35% and the PTA contribution is ~23%. Thus, the contribution to the HDLC selectivity due to the selective surfactant is ~ 10% (68% - 35% - 23%). The estimations for % selectivity contribution from each of the three components were calculated with various formulas. The surfactant testing reported in Table 7 used a formula with lower than optimum PTA and in a less accurate format. The surfactant contribution is the lowest possible estimate based on an over estimation of the PTA contribution used in this calculation.

EXAMPLE 5

Effect of PEG and Dimedone on HDLC Specificity

In another embodiment, other identified contributors refine the assay's accuracy as indicated by an analysis using human HDL and LDL test fluids. Using a PEG polymer and adding dimedone increase the assay's HDLC selectivity. For instance, PEG in the '-02' layer reduced the cross-reactivity of LDL by approximately 5% (17.5 - 12.8) compared to the IMnAg polymer (Table 8). In the presence of the PEG '-02' layer, the addition of dimedone reduced the cross-reactivity of LDL by approximately 6% (12.8% - 7.1%).

TABLE 8 - Effect of IMnAg, PEG, and Dimedone on the LDL Cross-Reactivity

| Receptor | Receptor | LDL Conc. (mg/dL) | Pred on HDL (mg/dL) | % Cross-Reactivity |
|---|---|---|---|---|
| PEG | No Dimedone | 75 | 9.62 | 12.8% |
| IMnAg | No Dimedone | 75 | 13.12 | 17.5% |
| PEG | Dimedone | 75 | 5.30 | 7.1% |

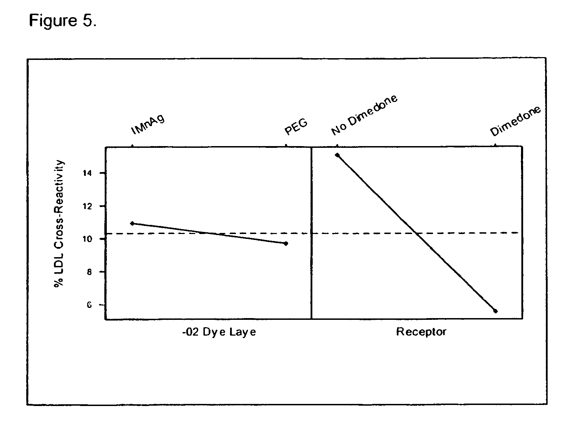

Another analysis used a balanced 2x2 design of experiments (DOE) test with the same factors (IMnAg & PEG in '-02', ± dimedone in '-03') and yielded similar results as shown in Table 8, except that the dimedone appeared to be a more important factor in LDL cross-reactivity than PEG. These results appear in Table 9. In this analysis, the PEG reduced the cross-reactivity of LDL by approximately 2%, while the dimedone reduced the cross-reactivity of LDL by approximately 10%. Utilizing the balanced 2x2 DOE, the main effects plot for LDL cross-reactivity was determined and appear in Figure 5. By this method, the contribution of the PEG to HDLC specificity is approximately 1 %, which is consistent with the initial results reported above and similar to the direct analysis method in Table 8. The dimedone has a larger contribution using the DOE analysis method (10%) and the contribution is slightly larger than previously estimated 5% contribution determined in the test fluid method. Because of the small errors involved in this estimation, the dimedone contribution to HDLC selectivity is in the 5% - 10% range.

TABLE 9 - Effect of PEG and Dimedone on the LDL Cross-Reactivity

| '-02' Dye Layer | Receptor | LDL Conc. (mg/dL) | Pred on HDL Web (mg/dL) | %Cross-Reactivity |
|---|---|---|---|---|
| 0.5 g/m² IMnAg | 0 g/m² Dimedone | 75 | 12.19 | 16.3% |
| 2.0 g/m² PEG | 0 g/m² Dimedone | 75 | 10.41 | 13.9% |
| 0.5 g/m² IMnAg | 0.5 g/m² Dimedone | 75 | 4.22 | 5.6% |
| 2.0 g/m² PEG | 0.5 g/m² Dimedone | 75 | 4.14 | 5.5% |

EXAMPLE 6

Summary of HDLC Selectivity

The preferred embodiment of the present invention includes three major identified factors influencing HDLC selectivity in the thin-film format, which are the non-HDL precipitant (PTA), the HDL selective surfactant, and the HDL selective CEH. Evaluating different coatings in the presence and absence of these three factors provides an estimate of the contribution of each factor to the overall assay HDLC selectivity. The total contribution to the HDLC specificity from major and minor contributors to selectivity is shown in Table 10. The less-than 100% recovery is most likely indicative of both the small errors in determining the contribution of each individual component from separate tests compared to one large Design of Experiments (DOE) containing all the factors simultaneously, and of the discrepancies between different formulas and formats to derive the contribution of the other components. Optimal, and acceptable, accuracy of the dry analytical element is conferred in the presence of all preferred HDL selective agents.

TABLE 10 - Contribution of different factors to the HDLC specificity

| Factor | %Contribution to HDLC Specificity |
|---|---|
| HDL Selective | 35% |
| Phosphotungstic | 23% |
| HDL Selective | 10% |
| Dimedone | 7% |
| PEG | 2% |
| Total Specificity | 77% |

EXAMPLE 7

PEG Improves Assay Precision and Dynamic Range

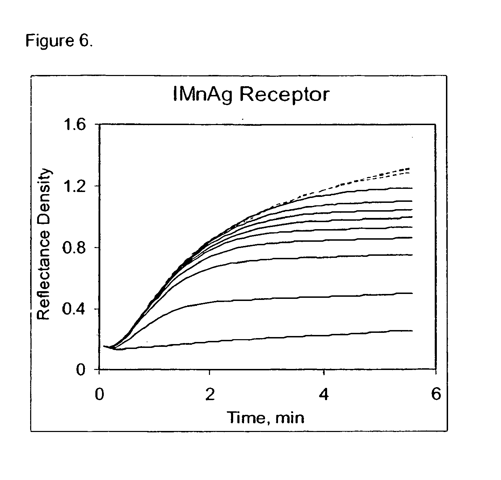

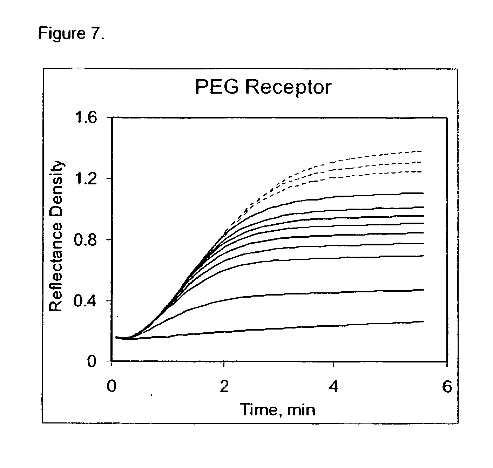

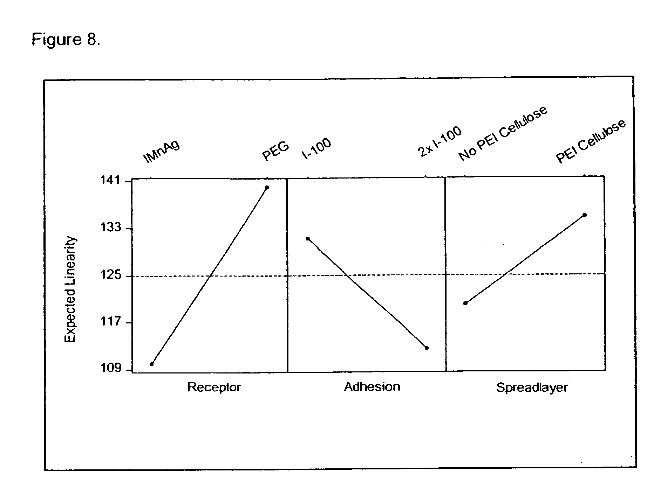

Although PEG in the '-03' layer enhanced HDLC specificity, the polymer also provided several other benefits to the assay. The first improvement appears directly in the human HDL test fluid kinetics (Figure 6). For the coating with IMnAg in the '-03' layer, the highest three concentration HDL fluids (dashed lines: 105 mg/dL, 120 mg/dL, & 150 mg/dL HDLC) show nearly identical kinetics as the high-end response levels off. In contrast, the formula with the PEG '-03' layer shows increasing response for these three high concentration HDL test fluids (Figure 7). This difference between the two formulae translates into better linearity and a higher dynamic range claim when PEG is used in the '-03' layer. The linearity assessment through the DOE analysis also shows a substantial main effect for linearity with PEG compared to the IMnAg polymer in the '-03' layer (Figure 8). The PEG in the '-03' layer adds approximately 30 mg/dL to the high end dynamic range compared to the IMnAg '-03' layer.

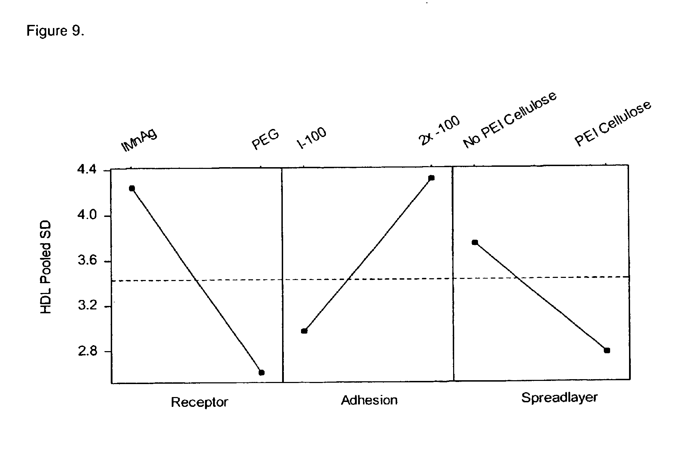

In addition to the dynamic range and linearity improvement with PEG in the '-03' layer, a large improvement in precision was also observed with the PEG-containing formula. In the presence of a nominal I-100 adhesion layer (0.44 g/m²), the imprecision was decreased by over 60% for the PEG-containing formula. Similar trends were observed with the 2x I-100 layer and the added PEI Cellulose, but the magnitude of the PEG precision improvement was less substantial. The DOE analysis of all three factors demonstrates that the PEG '-03' layer is a substantial factor for improved precision, but both the thinner I-100 and PEI Cellulose addition can also contribute significantly to improved precision (Figure 9).

However, the PEI Cellulose in the spread layer caused an undesired increase in LDL Cross-Reactivity. Based on the benefits observed in these tests for the PEG '-03' layer (precision, linearity, and kinetic endpoint), PEG was a preferred polymer choice for the direct HDL thin-film assay.

TABLE 11 - Pooled Standard Deviation Precision for the Human HDL & LDL Test Fluids

| Receptor | Adhesion | Spreadlayer | Pooled SD (HDL & LDL) | Pooled SD (HDL only) |
|---|---|---|---|---|
| 0.5 g/m$^2$ IMnAg | 0.44 g/m$^2$ I-100 | No PEI Cellulose | 4.04 | 4.65 |
| 4.0 g/m$^2$ PEG | 0.44 g/m$^2$ I-100 | No PEI Cellulose | 1.48 | 1.7 |
| 0.5 g/m$^2$ IMnAg | 0.88 g/m$^2$ I-100 | No PEI Cellulose | 4.47 | 3.5 |
| 4.0 g/m$^2$ PEG | 0.88 g/m$^2$ I-100 | No PEI Cellulose | 3.05 | 2.95 |
| 0.5 g/m$^2$ IMnAg | 0.44 g/m$^2$ I-100 | PEI Cellulose | 2.58 | 2.95 |
| 4.0 g/m$^2$ PEG | 0.44 g/m$^2$ I-100 | PEI Cellulose | 2.29 | 2.62 |

EXAMPLE 8

Use of Polymeric Matrices in the '-02' Layer Eliminates Interference from Hemolyzed Samples.

Formats using gelatin as the matrix for the '-02' dye-containing layer were found to predict aberrantly low HDLC values from serum samples in which hemolysis had occurred. This negative interference was caused by Catalase released from the lysed cells rather than from hemoglobin. Catalase interferes with the $H_2O_2$-mediated dye-formation step of the cholesterol enzyme cascade. Changing the matrix of the dye-containing '-02' layer eliminated Catalase interference. Serum samples spiked with various known amounts of a severely hemolyzed serum preparation (hemolysate) were evaluated on coatings containing IMnAg, PEG, or gelatin as the '-02' layer matrix. As detailed in Table 12, substituting the polymers IMnAg or PEG for gelatin in the -02 layer essentially eliminated any negative Catalase interference with samples spiked with hemolysate to 500 mg/dL hemoglobin.

TABLE 12 - Elimination of Hemolysate Interference through use of IMnAg or PEG in the Dye-containing '-02' layer.

| '-02' Layer matrix | % bias at hemolysate spike level (mg/dL hemoglobin) | | |
|---|---|---|---|
| | 100 | 300 | 500 |
| Gelatin | -20.3% | -28.4% | -30.2% |
| IMnAg | -0.3% | 0.9% | -1.1% |
| PEG | 0.9% | 0.0% | 0.9% |

EXAMPLE 9

Preferred Slide Structure

By incorporation of the above discoveries in the dry element, an accurate, precise, and rapid HDLC assay has been developed. A preferred embodiment of the invention is presented in Table 13 below.

TABLE 13: A Preferred Slide Structure

| Layer | Elements |
|---|---|
| -06 (washcoat) | **_Phosphotungstic acid_** (PTA, 3 g/m$^2$) |
| | MgCl$_2$ (1.7 g/m$^2$) |

(continued)

| Layer | Elements |
|---|---|
| '-05' (spread layer) | $BaSO_4$ (107.8 g/m$^2$) |
| | Xylene (33.59 g/m$^2$) |
| | Cellulose acetate (7.19 g/m$^2$) |
| | Dimethyl benzene (43.49 g/m$^2$) |
| | Acetone (101.85 g/m$^2$) |
| | Estane-polyurethane g/m$^2$) |
| | Emulgen B-66 (7 g/m$^2$) |
| '-04' (adhesion laver) | I-100 (0.435 g/m$^2$) |
| '-03' (polymer receptor layer) | *Candida rugosa* Lipase (13.4 kU/m$^2$) |
| | Cellulomonas Cholesterol Oxidase (8 kU/m$^2$) |
| | Polyethylene glycol MW 8000 (4 g/m$^2$) |
| | Zonyl FSN (0.01 g/m$^2$) |
| | TES (0.1 g/m$^2$ pH 7.0) |
| | Dimedone (0.5 g/m$^2$) |
| '-02' (dye sublayer) | TES (0.1 g/m$^2$, pH 7.0) |
| | Polyethylene glycol 8000 (2 g/m$^2$) or IMnAg (0.5 g/m$^2$) |
| | Leuco dye (0.2 g/m$^2$) |
| '-01' (gel layer) | Gel (6.458 g/m$^2$) |
| | BVSM hardener (2.3%) |
| | Ascorbic acid oxidase (3 kU/m$^2$) |
| | Horseradish peroxidase (22 kU/m$^2$) |
| | TES buffer (2.152 g/m$^2$, pH 7.0) |
| | Zonyl FSN (0.02 g/m$^2$) |
| (Note: top washcoat ('-06') layer is applied separately but is absorbed into the '-05' spread layer upon coating). It is to be understood that numerous changes and modifications may be made therein without departing from the scope and intent of the invention. For example, other useful formats are listed in Table 14. | |

TABLE 14 - Alternative Slide Structures ('-06' washcoat layer is contained in the '-05' spread layer)

A.  Spread Layer w/surfactant, PTA, $MgCl_2$
    Polymer Adhesion Layer
    Polymer Receptor Layer w/CEH, COD
    Sub Layer w/dye
    Gel Layer w/POD

B.  Spread Layer w/surfactant, PTA, $MgCl_2$
    Polymer Adhesion Layer
    Polymer Receptor Layer w/CEH, COD
    Sub Layer w/dye
    Gel Layer w/POD, dye

(continued)

| | |
|---|---|
| C. | Spread Layer w/surfactant, PTA, MgCl$_2$<br>Polymer Adhesion Layer<br>Polymer Receptor Layer w/CEH, COD, POD<br>Sub Layer w/dye<br>Gel Layer |
| D. | Spread Layer w/surfactant, PTA, MgCl$_2$<br>Polymer Adhesion Layer<br>Polymer Receptor Layer w/CEH, COD, POD<br>Sub Layer w/dye<br>Gel Layer w/dye |
| E. | Spread Layer w/surfactant, MES, PTA, MgCl$_2$<br>Polymer Adhesion Layer<br>Polymer Receptor Layer w/CEH, COD, POD<br>Sub Layer w/dye<br>Gel Layer |
| F. | Spread Layer w/surfactant, MES, PTA, MgCl$_2$<br>Polymer Adhesion Layer<br>Polymer Receptor Layer w/CEH, COD, POD<br>Sub Layer w/dye<br>Gel Layer w/dye |

EXAMPLE 10

Accuracy Tests

HDLC thin film slides were made using the formula and format described as the preferred slide structure above, using 7 g/m$^2$ EMULGEN B-66 as the HDLC-selective surfactant, *Candida rugosa* lipase or Denka CEH as the HDL selective cholesterol ester hydrolase, and IMnAg or PEG as the -02 matrix. Accuracy versus the VITROS Magnetic HDLC precipitation method and pooled precision were evaluated using 30 patient samples. Results can be found in Table 15. The results show that this assay has acceptable accuracy and precision and is free from significant interference from hemolysed patient samples.

TABLE 15 - Precision, Accuracy, and Hemolysate Interference Results from a Dry Analytical Element Assay for HDLC Using the Preferred Format.

| -02 Matrix | CEH Source | Patient Accuracy | | | Precision | Interference |
|---|---|---|---|---|---|---|
| | | Slope | Intercept | Sy.x | Pooled SD | Hemolysate |
| PEG | Denka | 0.99 | 0.50 | 2.2 | 1.30 | >500 mg/dL Hb |
| IMnAg | *Candida rugosa* | 0.99 | 0.52 | 2.46 | 3.89 | >500 mg/dL Hb |

Literature Cited

1 Burstein, M., Scholnick, H.R., & Morfin, R. J Lipid Res, 1970, 11, 583-595.

2 Fredrickson, D.S., Levy, R.I., & Lindgren, F.T. J Clin Invest 1968, 47, 2446-2457.

3 Mayfield, C., Warnick, G.R., & Albers, J.J. Clin Chem 1979, 25, 1309-1313.

4 Burstein, M., & Scholnick, H.R. Adv Lipid Res 1973, 11, 67-108.

5 Briggs, C., Anderson, D., Johnson, P., & Deegan, T. Ann Clin Biochem 1981, 18, 177-181.

6 Kakuyama, T., Kimura, S., & Hasiguchi, Y. Clin Chem 1994, 40, A1104.

7 Nauck, M., März, W., & Wieland, H. Clin Chem 1998, 44, 1443-1451.

8 Arranz-Pena, M., Tasende-Mata, J., & Martin-Gil, F.J. Clin Chem 1998, 44, 2499-2505.

9 Yamamoto, M., Nakamura, M., Hino, K., Saito, K., & Manabe, M. Clin Chem 2000, 46, A98.

10 Matsui, H., Ito, Y., Ohara, S., & Fujiwara, A. *European Patent* EP 0887422A1 , Priority Date 09/12/1996.

11 Hino, K., Nakamura, M., & Manabe, M. *United States Patent* 5,773,304, Priority Date 1/31/1995.

12 Izawa, S., Okada, M., Matsui, H., & Horita, Y. J Med Pharm Sci 1997, 37, 1385-1388.

13 Sugiuchi, H., Uji, Y., Okabe, H., Irie, T., Uekama, K., Kayahara, N., & Miyauchi, K. Clin Chem 1995, 41, 717-723.

14 Przybylowicz, E.P., & Millikan, A.G. *United States Patent* US 3,992,158 A, Priority Date 01/02/1975.

15 Pierce, Z.R., & Frank, D.S. *United States Patent* US 4,357,363 A, Priority Date 11/20/1981.

16 Pierce, Z.R., & Frank, D.S. *United States Patent* US 4,258,001 A, Priority Date 12/27/1978.

17 Burnstein, M., Samaille, J. Clin. Chim. Acta 1960, 5, 609.

18 Wilson, D. E., & Spizer, M. J. J. Lab. Clin. Med. 1973, 82, 473.

19 Vikari, J. Scand. J. Clin. Lab. Invest. 1976, 36, 265.

**Claims**

1.  A dry analytical element for the quantification of high-density lipoprotein cholesterol comprising:

    (A) a support; and
    (B) one or more additional layers comprising:

    1) phosphotungstic acid, a non-high density lipoprotein precipitant;
    2) a high-density lipoprotein selective surfactant; and
    3) a high-density lipoprotein selective cholesterol ester hydrolase enzyme.

2.  The analytical element of claim 1, wherein the one or more additional layers further comprises polyethylene glycol.

3.  The analytical element of claim 1 or claim 2, wherein the one or more additional layers further comprises dimedone (5,5-dimethyl-1,3-cyclohexanedione)

4.  The dry analytical element of claim 1, comprising a support with multiple layers coated in the following order, top down to the support:

    a. a washcoat layer comprising phosphotungstic acid, a non-high-density lipoprotein precipitant;
    b. a porous spread layer comprising a high-density lipoprotein selective surfactant;
    c. an adhesion layer;
    d. a polymer receptor layer comprising a high-density lipoprotein selective cholesterol ester hydrolase enzyme:

e. a dye sublayer comprising a polymer; and

f. a gel layer comprising binder materials.

**5.** The analytical element of claim 4, wherein the polymer receptor area comprises polyethylene glycol.

**6.** The analytical element of claim 4 or claim 5, wherein the dye sublayer comprises polyethylene glycol or polyacrylamide co-polymer.

**7.** The analytical element of any one of claims 4 to 6, wherein the polymer receptor layer comprises dimedone (5,5-dimethyl-1,3-cyclohexanedione),

**8.** The analytical element of any one of claims 4 to 7, wherein the dye layer or the gel layer comprises a leuco dye (2-(3,5-dimethoxy-4-hydroxyphenyl)-4,5-bis-(4-dimethylaminophenyl)imidazole.

**9.** The analytical element of any one of claims 4 to 8, wherein the gel layer comprises horseradish peroxidase.

**10.** The analytical element of any one of claims 4 to 9, wherein the polymer receptor layer comprises cholesterol oxidase from Cellulomonas.

**11.** The analytical element of any one of claims 1 to 10, wherein the high-density lipoprotein selective surfactant in EMULGEN B-66 (a polyalkylene derivative) or EMULGEN A-90 (a polyalkylene derivative).

**12.** The analytical element of any one of claims 1 to 11, wherein the high-density lipoprotein selective cholesterol ester hydrolase enzyme is *Candida rugosa* lipase.

**13.** The analytical element of any one of claims 1 to 12, wherein the support comprises transparent polystyrene, polyester, polycarbonate or cellulose ester.

**Patentansprüche**

**1.** Trockenanalyseelement zur Quantifizierung von HDL(High-Density-Lipoprotein)-Cholesterin, umfassend:

(A) einen Träger; und
(B) eine oder mehrere zusätzliche Schichten, umfassend:

1) Wolframatophosphorsäure, ein Nicht-HDL-Fällmittel;
2) ein HDL-selektives Tensid; und
3) ein HDL-selektives Cholesterinester-Hydrolase-Enzym.

**2.** Analyseelement nach Anspruch 1, wobei die eine oder mehreren zusätzlichen Schichten ferner Polyethylenglykol umfassen.

**3.** Analyseelement nach Anspruch 1 oder Anspruch 2, wobei die eine oder mehreren zusätzlichen Schichten ferner Dimedon (5,5-Dimethyl-1,3-cyclohexandion) umfassen.

**4.** Trockenanalyseelement nach Anspruch 1, umfassend einen Träger mit mehreren Schichten, die in der folgenden Reihenfolge von oben nach unten zum Träger hin aufgetragen sind:

a. eine Washcoat-Schicht, umfassend Wolframatophosphorsäure, ein Nicht-HDL-Fällmittel;
b. eine poröse Spreitschicht, umfassend ein HDLselektives Tensid;
c. eine Haftschicht;
d. eine Polymerrezeptorschicht, umfassend ein HDLselektives Cholesterinester-Hydrolase-Enzym;
e. eine Farbstoffunterschicht, umfassend ein Polymer; und
f. eine Gelschicht, umfassend Bindemittelmaterial.

**5.** Analyseelement nach Anspruch 4, wobei der Polymerrezeptorbereich Polyethylenglykol umfasst.

**6.** Analyseelement nach Anspruch 4 oder Anspruch 5, wobei die Farbstoffunterschicht Polyethylenglykol- oder Polyacrylamid-Copolymer umfasst.

**7.** Analyseelement nach einem der Ansprüche 4 bis 6, wobei die Polymerrezeptorschicht Dimedon (5,5-Dimethyl-1,3-cyclohexandion) umfasst.

**8.** Analyseelement nach einem der Ansprüche 4 bis 7, wobei die Farbstoffschicht oder die Gelschicht einen Leukofarbstoff (2-(3,5-Dimethoxy-4-hydroxyphenyl)-4,5-bis-(4-dimethylaminophenyl)imidazol) umfasst.

**9.** Analyseelement nach einem der Ansprüche 4 bis 8, wobei die Gelschicht Meerrettich-Peroxidase umfasst.

**10.** Analyseelement nach einem der Ansprüche 4 bis 9, wobei die Polymerrezeptorschicht Cholesterin-Oxidase aus Cellulomonas umfasst.

**11.** Analyseelement nach einem der Ansprüche 1 bis 10, wobei das HDL-selektive Tensid in EMULGEN B-66 (einem Polyalkylen-Derivat) oder EMULGEN A-90 (einem Polyalkylen-Derivat).

**12.** Analyseelement nach einem der Ansprüche 1 bis 11, wobei es sich bei dem HDL-selektiven Cholesterinester-Hydrolase-Enzym um Lipase aus *Candida rugosa* handelt.

**13.** Analyseelement nach einem der Ansprüche 1 bis 12, wobei der Träger durchsichtiges bzw. durchsichtigen Polystyrol, Polyester, Polycarbonat oder Celluloseester umfasst.


**Revendications**

**1.** Élément analytique sec pour la quantification du cholestérol des lipoprotéines de haute densité comprenant :

    (A) un support ; et
    (B) une ou plusieurs couches supplémentaires comprenant :

        1) de l'acide phosphotungstique, un non-précipitant des lipoprotéines de haute densité ;
        2) un tensioactif sélectif envers les lipoprotéines de haute densité ; et
        3) une enzyme cholestérol ester hydrolase sélective envers les lipoprotéines de haute densité.

**2.** Élément analytique selon la revendication 1, dans lequel la ou les couches supplémentaires comprennent en outre du polyéthylène glycol.

**3.** Élément analytique selon la revendication 1 ou la revendication 2, dans lequel la ou les couches supplémentaires comprennent en outre de la dimédone (5,5-diméthyl-1,3-cyclohexanedione).

**4.** Élément analytique sec selon la revendication 1, comprenant un support avec de multiples couches déposées dans l'ordre suivant, de haut en bas sur le support :

    a. une couche de revêtement réactif comprenant de l'acide phosphotungstique, un non-précipitant des lipoprotéines de haute densité ;
    b. une couche d'étalement poreuse comprenant un tensioactif sélectif envers les lipoprotéines de haute densité ;
    c. une couche d'adhérence ;
    d. une couche de récepteur polymère comprenant une enzyme cholestérol ester hydrolase sélective envers les lipoprotéines de haute densité ;
    e. une sous-couche de colorant comprenant un polymère ; et
    f. une couche de gel comprenant des matériaux liants.

**5.** Élément analytique selon la revendication 4, dans lequel la zone de récepteur polymère comprend du polyéthylène glycol.

**6.** Élément analytique selon la revendication 4 ou la revendication 5, dans lequel la sous-couche de colorant comprend du polyéthylène glycol ou un copolymère de polyacrylamide.

**7.** Élément analytique selon l'une quelconque des revendications 4 à 6, dans lequel la couche de récepteur polymère comprend de la dimédone (5,5-diméthyl-1,3-cyclohexanedione).

**8.** Élément analytique selon l'une quelconque des revendications 4 à 7, dans lequel la couche de colorant ou la couche de gel comprend un leuco-colorant (2-(3,5-diméthoxy-4-hydroxyphényl)-4,5-bis(4-diméthylaminophényl)-imidazole).

**9.** Élément analytique selon l'une quelconque des revendications 4 à 8, dans lequel la couche de gel comprend de la peroxydase de raifort.

**10.** Élément analytique selon l'une quelconque des revendications 4 à 9, dans lequel la couche de récepteur polymère comprend de la cholestérol oxydase de Cellulomonas.

**11.** Élément analytique selon l'une quelconque des revendications 1 à 10, dans lequel le tensioactif sélectif envers les lipoprotéines de haute densité dans l'EMULGEN B-66 (un dérivé de polyalkylène) ou l'EMULGEN A-90 (un dérivé de polyalkylène).

**12.** Élément analytique selon l'une quelconque des revendications 1 à 11, dans lequel l'enzyme cholestérol ester hydrolase sélective envers les lipoprotéines de haute densité est la lipase de *Candida rugosa.*

**13.** Élément analytique selon l'une quelconque des revendications 1 à 12, dans lequel le support comprend du polystyrène, polyester, polycarbonate ou ester de cellulose transparent.

Figure 1.

Beef Pancreas CEH

Figure 2.

Porcine Pancrease CEH

Figure 3.

Candida rugosa Lipase

Figure 4.

Figure 5.

Figure 6.

Figure 7.

Figure 8.

Figure 9.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 399215814 B
- US 435736315 B
- US 425800116 B
- EP 0887422 A1
- US 5773304 A
- US 3992158 A
- US 4357363 A
- US 4258001 A

**Non-patent literature cited in the description**

- **BURSTEIN, M. ; SCHOLNICK, H.R. ; MORFIN, R.** *J Lipid Res,* 1970, vol. 11, 583-595
- **FREDRICKSON, D.S. ; LEVY, R.I. ; LINDGREN, F.T.** *J Clin Invest,* 1968, vol. 47, 2446-2457
- **MAYFIELD, C. ; WARNICK, G.R. ; ALBERS, J.J.** *Clin Chem,* 1979, vol. 25, 1309-1313
- **BURSTEIN, M. ; SCHOLNICK, H.R.** *Adv Lipid Res,* 1973, vol. 11, 67-108
- **BRIGGS, C. ; ANDERSON, D. ; JOHNSON, P. ; DEEGAN, T.** *Ann Clin Biochem,* 1981, vol. 18, 177-181
- **KAKUYAMA, T. ; KIMURA, S. ; HASIGUCHI, Y.** *Clin Chem,* 1994, vol. 40, A1104
- **NAUCK, M. ; MÄRZ, W. ; WIELAND, H.** *Clin Chem,* 1998, vol. 44, 1443-1451
- **ARRANZ-PENA, M. ; TASENDE-MATA, J. ; MARTIN-GIL, F.J.** *Clin Chem,* 1998, vol. 44, 2499-2505
- **YAMAMOTO, M. ; NAKAMURA, M. ; HINO, K. ; SAITO, K. ; MANABE, M.** *Clin Chem,* 2000, vol. 46, A98
- **IZAWA, S. ; OKADA, M. ; MATSUI, H. ; HORITA, Y.** *J Med Pharm Sci,* 1997, vol. 37, 1385-1388
- **SUGIUCHI, H. ; UJI, Y. ; OKABE, H. ; IRIE, T. ; UEKAMA, K. ; KAYAHARA, N. ; MIYAUCHI, K.** *Clin Chem,* 1995, vol. 41, 717-723
- **BURNSTEIN, M. ; SAMAILLE, J.** *Clin. Chim. Acta,* 1960, vol. 5, 609
- **WILSON, D. E. ; SPIZER, M. J.** *J. Lab. Clin. Med.,* 1973, vol. 82, 473
- **VIKARI, J.** *Scand. J. Clin. Lab. Invest.,* 1976, vol. 36, 265